# EUROPEAN PATENT APPLICATION

(11) **EP 2 119 403 A1**
(43) Date of publication of application: **18.11.2009**
(21) Application number: 09160397.7
(22) Date of filing: 15.05.2009
(51) Int. Cl.: A61B 17/32, A61B 17/00

(54) **Osteotomes for ultrasonic bone surgery, particularly maxillofacial, dental and orthopedic surgery**

(30) Priority: 16.05.2008 IT TO20080369
(71) Applicant: Blus, Cornelio, 14030 Montemagno (IT)
(72) Inventor: Blus, Cornelio, 14030 Montemagno (IT)
(74) Representative: Fioravanti, Corrado

(57) **Abstract**

The osteotome (10) serves to cut bone tissues using vibrations in the ultrasonic field, and comprises an elongated body made of steel or Titanium alloy which forms a rectilinear tang (20), a cylindrical or truncated-cone shaped end head (30) tapered to a free end thereof, and an intermediate connecting portion (40). This portion includes a proximal rectilinear section (41) aligned with the tang (20), and a sinuous section (42), evenly joined to the rectilinear section (41) and head (30). The sinuous section (42) forms two subsequent bends with concavities facing opposite directions.

## Description

The present invention relates to osteotomes to be used in ultrasonic bone surgery. The osteotomes according to the invention are intended to be applied, particularly, though not exclusively, in the fields of orthopedics, neurosurgery, otolaryngology surgery, maxillo-facial, dental and veterinary surgery.

The ultrasonic technique is widespread in bone surgery, where it is widely used also because it does not cause any undesired cutting of soft tissues. Surgical instruments are known to contain piezoelectric transducers, which, when electrically powered, vibrate a metal tip that can cut the bone or cause the separation of biological tissues. See, for example, patent publications US-2 984 241, US-4 188 952, EP-0 238 667, WO 2008/012359.

The object of the invention is to provide an osteotome allowing to cut and pierce the bone tissue in a particularly accurate, easy manner, thereby minimizing the risk of damaging tissues close to those to be operated.

This and other objects and advantages are achieved, according to the present invention, by an osteotome for ultrasonic surgery having the features as defined in the annexed claims.

The advantages of the invention will be understood from the reading of the description below, which is given as a non-limiting example with reference to the annexed drawings, in which:
Fig. 1, 2 and 3 are a perspective view, a rear view and a side view, respectively, of a first embodiment of an osteotome according to the invention;
Fig. 4 is a partial view, in an enlarged scale, of a head end of a second embodiment of the osteotome;
Fig. 5 is a perspective view of a third embodiment of the osteotome;
Fig. 6 is an enlarged view of a detail in Fig. 5;
Fig. 7 is a perspective view of a fourth embodiment of the osteotome;
Fig. 8 is an enlarged view of a detail in Fig. 7; and
Fig. 9 and 10 are a perspective view and a side view of a fifth embodiment of an osteotome according to the invention.

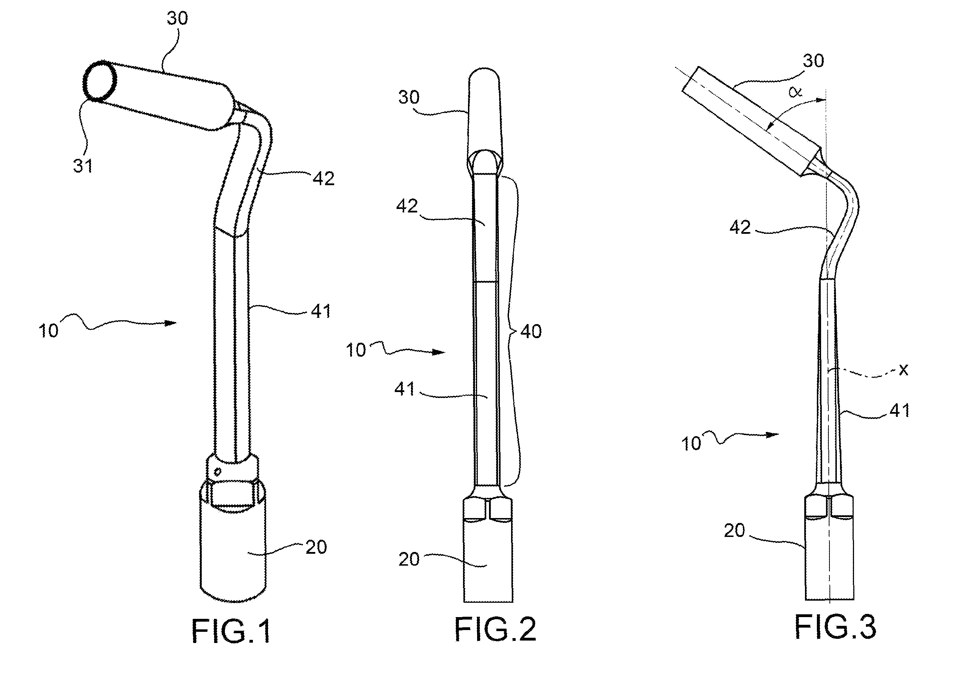

A first embodiment of an osteotome for ultrasonic surgery according to the invention, as shown in Fig. 1-3, is generally designated with 10. The osteotome 10, consisting of an elongated body made of stainless steel for surgical use or a Titanium alloy, preferably grade 5 Titanium, is extended along a central axis x which has a sequence of rectilinear and bent sections laying in a same plane. The osteotome comprises a rectilinear proximal section or locking tang 20, an operating head 30 located on the free end side of the osteotome, and an elongated intermediate portion 40 connecting the tang to the head.

The cylindrical tang 20, being of a greater thickness than the other parts of the osteotome, is intended to be fitted or screwed or otherwise securely mounted to a cylindrical handpiece (not shown) acting as a handle for the surgeon. The handpiece is part of an ultrasonic equipment which electrically supplies a series of piezoelectric transducers housed in the handpiece, such as to vibrate the osteotome at a frequency comprised within the ultrasonic field. The manufacturing and operating characteristics of the above-said type of ultrasonic equipment are not relevant per se to the purposes of the understanding of the invention and accordingly they will not be described in detail herein. It will be sufficient to remember herein that this equipment is adjustable as relates to the vibration frequency imparted to the tip and the input power.

The intermediate connecting portion 40 comprises a more proximal rectilinear section 41 aligned with the tang, of which it can be seen as a thinner extension, evenly joined to a more distal sinuous section 42, substantially S-shaped or question mark ("?")-shaped, which is joined to the operating head 30 thereby forming two successive bends with concavities facing opposite directions. The connecting portion 40, and particularly the sinuous section 42, is progressively flattened, and becomes thinner towards the head 30.

The head 30 has a generally cylindrical or weakly truncated-cone shape, slightly tapered to the free end 31 of the osteotome. Due to the sinuous section 42, the angle α formed between the head axis 30 and tang axis ranges between about 45° and about 70°.

Ultrasonic vibrations are imparted to the tang 20 in the form of axial pulses, and are propagated and amplified through the intermediate section 40 and transmitted to the end head 30 and cutting end 31 thereof which is brought in contact with the bone tissue to be cut or pierced.

Due to the above-described configuration, as well as the selection of the 5 grade titanium as the ideal material for the transmission of the vibrations, these osteotomes lend themselves to cut and pierce bone tissues in an optimum manner when applied to an ultrasonic equipment of the above-mentioned type under certain operating conditions, which will be described below.

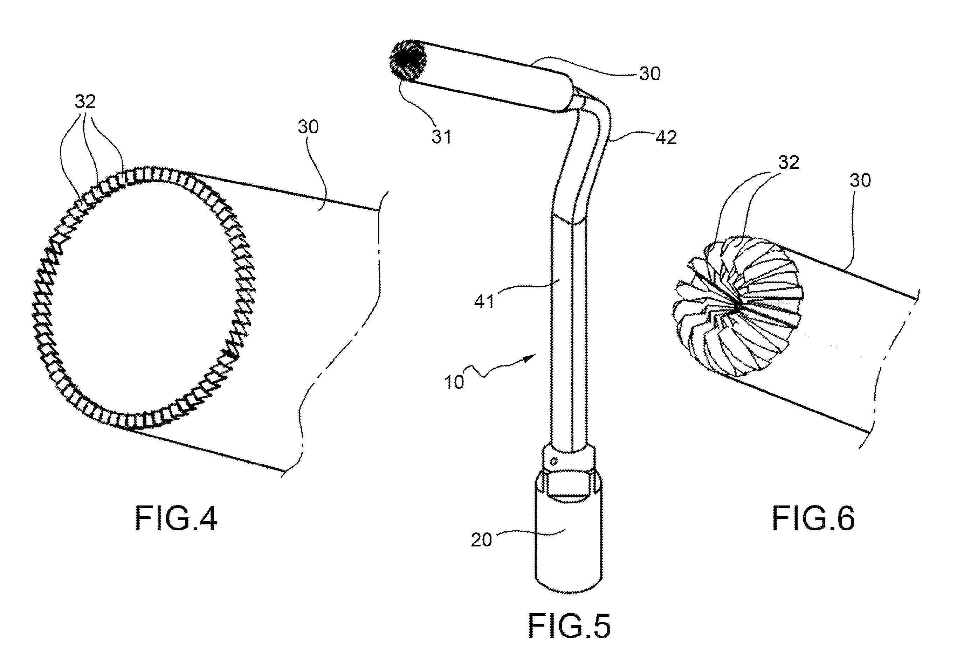

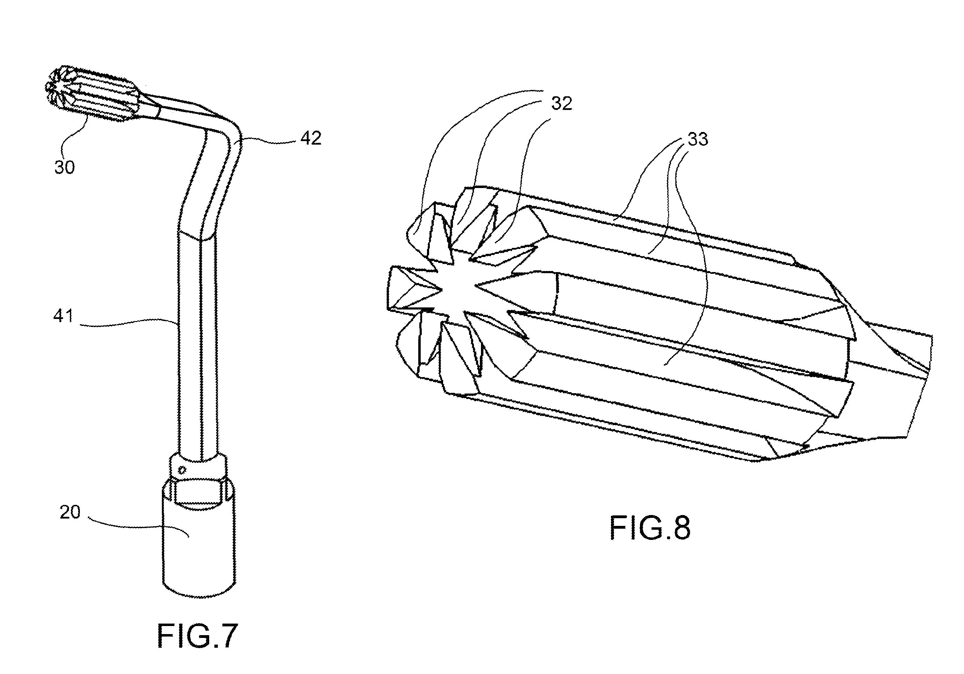

From experimental uses, it has resulted to be particularly easy for the surgeon to make rectilinear cuts and holes of a considerable accuracy due to the generally cylindrical or weakly truncated-cone shape of the head 30, which facilitates maintaining the instrument in the axial feeding direction. Particularly surprising results, relative to the osteotome penetration capacity, are obtained when the forward face has a series of radially oriented relieves 32. These relieves can be arranged on the circular edge of the forward face when the head 30 is of a tubular type (Fig. 1 and 4), or can be radially distributed substantially throughout the forward or front face of the head (Fig. 5 and 6). Preferably, the radial relieves have acute angle vertexes which converge to the center of the forward end of the head 30. The capacity of penetration in the bone is enhanced by the optional provision of longitudinal grooves or relieves 33 (Fig. 7 and 8) which are obtained on the cylindrical or truncated-cone surface of the head 30, they are preferably provided as extensions of the front recesses or relieves 32. It has been appreciated that the osteotome behavior, despite the demonstrated easiness of penetration thereof, has revealed little traumatic both for the bone and soft tissues (mucosa, membranes, nerves) contacted by the osteotome.

These surprising performance and results can be all obtained provided that the osteotome is vibrated within a frequency range between about 24 kHz and about 36 kHz, using on the equipment an input power ranging between about 20-90 W, and particularly about 42-90 W, with a sinusoid type wave.

The osteotomes according to the invention lend themselves in an optimum manner to the preparative operations of the implant site in all variants thereof, i.e. both for a normal implant site, and in the so-called post-extractive implantology and in the so-called technique of mini-elevation of the maxillary sinus by the crest route. In the latter technique, which is aimed at providing a vertical bone increase to firmly accommodate a dental implant, a through hole requires to be made in the maxillary crest and the Schneider membrane is to be elevated, and left intact, to make the *chip* bone graft, according to modes known to those skilled in the bone surgery art.

It was appreciated that, especially during the preparation of an implant site, the osteotomes according to the invention allow easily providing deep holes, by having access to areas which are usually difficult to be reached using traditional tips. In post-extractive implantology, the osteotomes herein allow correcting the direction of the hole, which is normally prevented by the hardness of the alveolar cortical bone. Based on the test results observed so far, the Applicant believes that these osteotomes, due to the geometry and delivered power thereof according to the above-said modes, enhance the cavitation effect which determines, with a particular effectiveness, the destruction of suspended bacteria where operations are carried out in areas infected by bacteria.

The studies that have been carried out so far have pointed out that, in the above-said operative conditions, an osteotome head having the physical-mechanical characteristics described above described a complex three-dimensional sinuous path that is given by the composition of individual vibrational modes on three orthogonal axes and that can vaguely resemble an "8".

The above-said results have been obtained with osteotomes provided by means of stock removal processing from a solid piece. The same osteotomes are then subjected to a thermal normalization process, to be capable of maintaining unchanged the molecular properties of the titanium alloy. It is then believed that this processing and this treatment give the osteotome better elastic properties, in terms of a greater vibration amplitude of the end head.

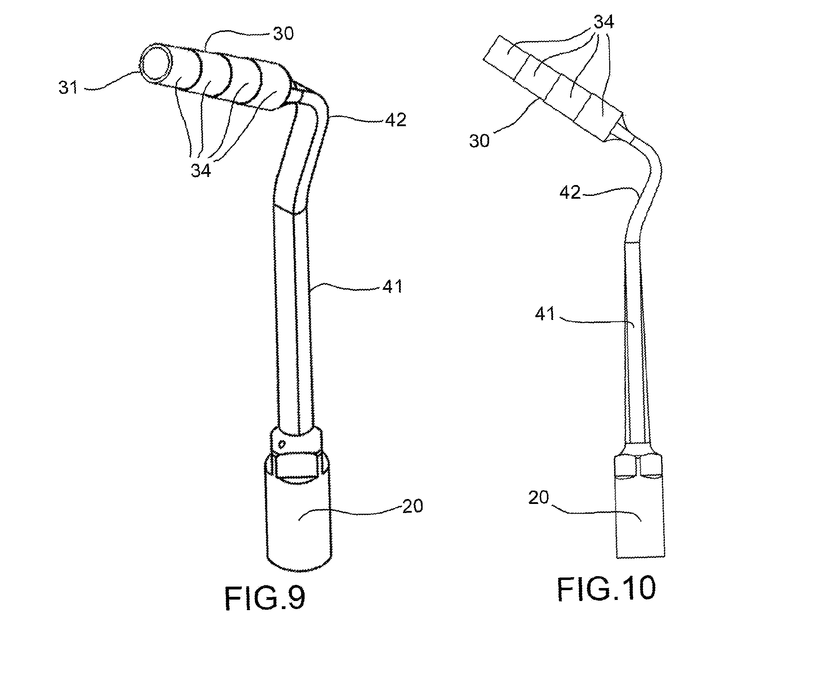

In the embodiment in Fig. 9 and 10 the generally truncated-cone shaped side surface of the head 30 consists of a sequence of cylindrical sections 34 having the same length and a gradually decreasing diameter in the direction of the free end 31, which besides producing a progressive enlargement of the perforation, give the surgeon an immediate perception of the depth of penetration into the bone.

It should be understood that the invention is not limited to the embodiments illustrated and described herein, which should be considered as exemplary embodiments of the osteotome; in fact, the invention is susceptible of modifications relative to shapes, size, and constructive details.

By way of a non-limiting example, in a small-sized tip, the truncated-cone shaped head 30 can have a diameter decreasing from 2,2 mm at the proximal end thereof, to 1,8 mm at the free or distal end 31 thereof, developing by a length of about 8-13 mm. In a greater size tip, with a head about 15-16 mm long, the head diameter decreases from 4,5 mm at the proximal head to 4,0 mm at the free distal end.

## Claims

1. An osteotome (10) for cutting bone tissues using vibrations in the ultrasonic field, **characterized in that** it comprises an elongated steel or titanium-alloy body which forms:
- a rectilinear tang (20) suitable to be locked to a surgical instrument capable of imparting vibrations at frequencies ranging between about 24 kHz and about 36 kHz to the osteotome (10);
- a substantially cylindrical or weakly truncated-cone shaped end head (30) tapered towards the head free end, wherein the head extends along an axis which forms with the axis of the tang an angle (α) ranging between about 45° and about 70°;
- a connecting portion (40), intermediate between the head (30) and the tang (20), comprising
- a proximal rectilinear section (41) aligned with the tang (20), and
- a sinuous section (42), evenly joined to the rectilinear section (41) and head (30), wherein the sinuous section (42) forms two successive bends with concavities facing in opposite directions.

2. The osteotome according to claim 1, **characterized in that** the end head (30) has a front surface (31) with a plurality of frontally projecting and radially extending relieves or ridges (32).

3. The osteotome according to claim 2, **characterized in that** the head (30) has a generally tubular shape and the radial relieves or ridges (32) are located on the free end edge (31) of the head.

4. The osteotome according to claim 2, **characterized in that** the radial relieves or ridges (32) are radially distributed substantially throughout the front surface or face of the head (30).

5. The osteotome according to claim 2 or 4, **characterized in that** the radial relieves or ridges (32) have acute angle vertexes converging to the center of the forward end of the head (30).

6. The osteotome according to any one of the preceding claims, **characterized in that** it comprises longitudinal grooves or relieves (33) obtained on the cylindrical or truncated-cone shaped surface of the head (30).

7. The osteotome according to any one of the preceding claims, **characterized in that** it comprises longitudinal grooves or relieves (33) obtained on the cylindrical or truncated-cone shaped surface of the head (30) axially aligned with the peripheral parts of relieves or ridges (32) frontally projecting and radially extending on a front surface (31) of the head (30).

8. The osteotome according to claim 1, **characterized in that** the sinuous section (42) is progressively thinned towards the head (30).

9. The osteotome according to claim 1, **characterized in that** the proximal rectilinear section (41) is progressively thinned towards the head (30).

10. The osteotome according to claim 1, **characterized in that** the head (30) has a generally truncated-cone shaped surface (30) comprising a sequence of cylindrical sections (34) of equal length and diameter gradually decreasing towards the free end (31) of the head.
